# EUROPEAN PATENT SPECIFICATION

(11) **EP 1 360 488 B1**
(45) Date of publication and mention of the grant of the patent: **13.04.2016**
(21) Application number: 01960173.1
(22) Date of filing: 12.07.2001
(51) Int. Cl.: G01N 33/536, G01N 33/569

(54) **SPATIALLY RESOLVED ENZYME-LINKED ASSAY**
ENZYMGEBUNDENER ASSAY MIT RÄUMLICHER AUFLÖSUNG
EPREUVE BIOLOGIQUE LIEE A UNE ENZYME A RESOLUTION SPATIALE

(30) Priority: 26.07.2000 DK 200001137; 29.09.2000 DK 200001446; 25.04.2001 DK 200100653
(43) Date of publication of application: 12.11.2003
(73) Proprietor: ChemoMetec A/S, 3450 Allerød (DK)
(72) Inventor: GLENSBJERG, Martin, DK-2700 Broenshoej (DK)
(74) Representative: Høiberg A/S
(86) International application number: PCT/DK2001/000490
(87) International publication number: WO 2002/008754

(56) References cited:
- WO-A-00/28297
- WO-A-89/06226
- WO-A-93/01308
- WO-A-98/59233
- GONZALEZ-GIL S ET AL: "DETECTION AND QUANTIFICATION OF ALKALINE PHOSPHATASE IN SINGLE CELLS OF PHOSPHORUS-STARVED MARINE PHYTOPLANKTON", MARINE ECOLOGY PROGRESS SERIES, INTER-RESEARCH, OLDENDORF, DE, vol. 164, 9 April 1998 (1998-04-09), pages 21-35, XP008001597, ISSN: 0171-8630
- CANO R J ET AL: "DNA HYBRIDIZATION ASSAY USING ATTOPHOS A FLUORESCENT SUBSTRATE FOR ALKALINE PHOSPHATASE", BIOTECHNIQUES, vol. 12, no. 2, 1992, pages 264-266, 268, XP009175271, ISSN: 0736-6205

## Description

The present invention relates to a method of assessing at least one quality parameter and/or at least one quantity parameter of at least one cell wherein said at least one cell is connected to a catalyst capable of catalysing a substrate into a product, whereby the cell is assessed through detection of product produced around the cell.

### Background

Detection of a substance or a particle using staining of the substance or particle to aid detection is widely used. However, many substances and particles are so small that although stained it is difficult to detect them without using very high magnification increasing the requirements to the equipment used.

A classical amplification technique is that of enzyme-linked assay. A ligand reacting specifically with the analyte is bound to an enzyme, and after excess ligand-enzyme is removed, the analyte-ligand-enzyme complex is detected by reaction with a chromogenic substrate, a colourless material which is acted upon by the enzyme to form a coloured product. Because of its large amplification factor, enzyme-linked assays offer high sensitivity, and are particularly useful for detection of small amounts of antigens.

In traditional enzyme-linked assays (ELISA) the amplification increases sensitivity at the expense of precision, because amplification factors are not exact. It is not possible to distinguish a strong reaction from a few molecules from a weak reaction from a high number of molecules.

An attempt to overcome some of the problems related to preciseness detection is using ELISPOT or in situ ELISA or spot-ELISA, that independent of the name relates to the use of ELISA technique to detect localised coloured spots on a solid support.

ELI-SPOT analysis allows the detection and enumeration of particles, such as virus or polypeptide producing cells or antigen-presenting cells. The spots produced may be enumerated by the use of a microscope or by use of video-imaging. The principle of ELI-SPOT is for example described in Sedgwick, J.D. and Holt, P.G. " A Solid - Phase Immunoenzymatic Technique for the Enumeration of Specific Antibody-Secreting Cells, Journal of Immunological Methods, 57 (1983) 301-309, wherein secreted antibodies to be detected are captured by antigens attached to a fixed solid support. The secreted antibodies are detected by adding antibody-enzyme complexes, said antibodies having specificity towards the secreted antibodies. The substrate is added in a warm agarose gel, that is allowed to hardened whereupon spots of insoluble product may be detected in the hardened agarose gel using a microscope.

Gonzalez-Gil et al, 1998, (Marine Ecology Progress Series, vol 164, 21-35) teaches two different substrates to detect alkaline phosphatase activity in single cells of marine phytoplankton. According to the first method (ELF), the endogenous alkaline phosphatase produces an insoluble fluorescent product, which precipitates on the surface of the cells. In the other case the substrate is converted into a soluble product. According to Gonzalez-Gil et al, only the first method can be used for imaging of cells while in the second method the fluorescent product is distributed throughout the microwell.

### Summary of the invention

The present invention relates to a method for assessing at least one quality parameter or at least one quantity parameter of at least one species of cells in a liquid sample comprising the steps of
establishing a sample compartment having at least one wall,
arranging catalyst-cell complexes between the at least one species of cells and at least one catalyst in the sample compartment in a manner allowing the cells to move relative to the wall(s) of the sample compartment,
arranging a substrate in the sample compartment, said substrate being capable of being converted into a product through catalysing by said catalyst,
contacting the substrate with the catalyst-cell complexes of individual cells allowing a detectable amount of product spots to be produced, the product diffusing from the cells to form localised substantially spherical spots around the cells,
recording an image of the product spots related to individual cells in the sample compartment after a detectable amount of product has been produced and before the various spots become confluent, and
correlating the image to the at least one quality parameter or at least one quantity parameter of the at least one species of cells.

The spot produced from the product relates to one or a few cells, allowing an assessment of the parameter relating to the cells to take place. The three-dimensional formation of product leads to a more distinct identification of the cells relative to the background. The image of such a spot is in many aspects similar to an image of particles encountered in numerous other applications. It is therefore understood that substantially same techniques and methods can be used in the recording, processing and analysing an image of spots as would be used for particles.

As opposed to traditional ELI-SPOT technique the cells to be detected are not coupled to a solid support fixed to the sample compartment but may be positioned anywhere in the sample compartment during the contact between the substrate and cell-catalyst-complexes. The cells are capable of moving relative to the wall(s) of the sample compartment at least until the substrate is introduced into the sample compartment. Thereby contact between the cell-catalyst-complexes and the substrate is conducted more easily.

The complex between cell and catalyst may be formed in one or more of several ways, such as:
Through a linkage, wherein the term linkage means that the cell and catalyst are bound to each other.

For example, the cell-catalyst complex may be formed via a species-selective linkage, such as an immunological binding, i.e. the linkage formed between an antibody and its antigen.

Also the linkage may be through conjugation, i.e. the covalent binding between two compounds, for example between an enzyme and an antibody, an enzyme and avidin, an antibody and biotin.

In another embodiment the complex between the cell and the catalyst is formed through a production of catalyst in or adjacent the cell such as expression of an enzyme from a cell. The enzyme is located adjacent the cell after expression and an cell-catalyst complex is formed.

The correlation of the image to the at least one quality parameter or at least one quantity parameter of at least one species of cells in a sample preferably comprises estimation of the number of spots on the image and/or estimation of the size of spots on the image.

The method may be conducted in a system comprising
a device comprising a sample compartmentcomprising an exposing domain, an inlet through which a volume of a liquid sample representing the cell material can been introduced, and a flow system comprising at least a channel allowing at least a portion of the volume of the liquid sample to flow within the device,
the device further comprising means to control the flow of liquid around a catalyst-cell complex in the sample compartment, a detection device comprising at least a first detector for quantitatively detecting spatial image data and a processor for processing the detected image presentation,
the device and the detection device having means for arranging the device in relation to the detection device in a manner allowing electromagnetic signals from a sample in the exposing domain of the device to pass to the detection device and to form, in the detection device, a spatial image representation of the exposing domain.

In particular the flow system additionally comprises a compartment or a flow channel part in or from which at least part of one or more reaction components initially loaded in the compartment or flow channel part is added to at least a portion of the volume of the liquid representing the sample.

Furthermore, the system may comprise at least a first light source, which advantageously emits excitation light. The detector adapted to detect the fluorescence emitted from the sample is advantageously located on the same side of the exposing domain as the light source.

Also disclosed is the use of a system for diagnosis of a condition in an individual, such as the diagnosis of cardial infarct.

### Drawings

Fig. 1 shows a one sided excitation system.
Fig. 2 shows a cross-section of the excitation light filter in a plane parallel to the sample plane.
Fig. 3 shows the collection angle C and the angle E between the excitation main light path and the detection -sample axis.
Fig. 4 shows a double-sided excitation/detection system.
Fig. 5 shows a double-sided excitation system.
Fig. 6 shows a double-sided detection system.

### Detailed description of the invention

### Amplification of signal

The cell is detectable due to the amount of product being formed around the cell. Depending on the physical constraints in the sample compartment the product will diffuse from the cells to form localised substantially spherical spots around the cells or clusters of cells. The spot will increase with time due to the transport of product in the media generally through diffusion. Thus it is possible to monitor the formation of the spots growing with time both with regard to intensity and size.

### Parameter

A quantity parameter according to the invention is the number of cells present in the sample and/or the concentration of cells in the sample, whereas quality parameter is information regarding viability, dead and/or dying organism including apopotosis, size, identity, respiration, presence, and morphology.

### Kinetics

The rate of formation of the product and thus the rate of change in the recorded image is dependent on the chemical and/or physical properties of the media in the sample compartment. In situations where the flow is limited this will often be defined by the rate of diffusion of substrate towards the catalytic site and/or the rate of diffusion of the product away from the catalytic site.

The image recorded of the product spots has to be recorded after a detectable amount of product has been produced and before the various spots become confluent, thereby inhibiting recording of an image. The image may be correlated to the at least one quality parameter or the at least one quantity parameter of the cell.

The time period from contacting the cell-catalyst-complexes with the substrate to recording the spots is mainly depending on the diffusion rate of substrate as well as product and on the kinetics of the process. The diffusion rate and the kinetics of the process may be controlled as discussed below.

The development of detectable spots is preferably observed within 60 minutes from contacting the cell-catalyst-complexes with the substrate. In more preferred embodiments the step of producing a product is below 15 minutes, preferably below 5 minutes, more preferably below 1 minute, more preferably below 30 seconds, more preferably below 15 seconds, more preferably below 10 seconds, more preferably below 5 seconds, more preferably below 2 seconds.

In a preferred embodiment the sample compartmentis exposed to the detection means before any development of detectable spots is commenced and at least one other exposure is conducted when sufficient time to obtain the developed spots have been reached.

By changing the viscosity of the environment in the sample compartment it is also possible to control the period of time of producing the product. The step of producing a product may be carried out in a liquid environment, or in a viscous environment. In the latter case, the viscosity may be adjusted to the substrate and catalyst used. Also, the step of producing a product is carried out in a semi-solid environment, preferably where the semi-solid environment is a gel. The semi-solid environment is preferably formed after the cells have been introduced to the sample compartment, preferably where the forming of the semi-solid environment is controlled by external factors such as temperature, light and agitation.

### Cells

The cell may be any cell capable of forming an cell-catalyst complex. As discussed above the cell may be bound by the catalyst via a species-specific binding, such as via an antigen-antibody binding.

The cells are preferably selected from mammalian cells, insect cells, reptile cells, fish cells, yeast cells, and fungi cells, more preferably from blood cells, sperm cells, and bone marrow cells.

The cells may be coupled to a solid support, such as beads, said beads being capable of being suspended in the sample compartment. The beads may be polymer beads. Often the polymer beads can have physical and/or chemical properties which can assist in the handling of the cell such as paramagnetic beads.

One embodiment of the present invention which involves the assessment of small cells is based on binding of the cell to a bead. This allows the cell and/ the cell-catalyst complex to be treated in a more simple manner during pre-treatment such as with centrifugation, filtration or magnetic separation.

The beads may be labelled themselves to improve accuracy in the identification of one or more cells. One embodiment of the present invention uses two or more types of beads which can have affinity to bind two or more different cells. If the cell-catalyst complex produces the same product regardless of which cell is involved, the identification of the polymer bead can be used to distinguish between which cell-catalyst complex produces the product the signal of which is detected. Such labelling of polymer beads can be based on colour, fluorescence, size or any other physical or chemical property.

Also, the cell may be a DNA or RNA containing cell whereby the DNA/RNA or a fraction thereof may be stained with a DNA staining compound. This is often preferred when a further confirmation or assessment of cell property or cell specificity is needed such as assessment of viability.

Apart from methods based on enzyme amplification (Enzyme Amplification Systems, EAS) other systems are also included in many preferred embodiments of the present invention. Among such specific amplification systems are the follows:
PAP: Peroxidase anti-peroxidase complex
APAAP: Alkaline phosphatase anti-alkaline phosphatase complex
BGABG: beta-galactosidase anti-beta-galactosidase complex
Cyclic conversion of NADH to NAD especially when the formation of NADH involves NADPH and Alkaline Phosphatase.

### Sample

The sample may be a liquid sample such as a sample selected from the group consisting of milk, milk products, urine, blood, sperm, nasal secrete, tears, faeces, waste water, process water drinking water, cerebro-spinal fluid, gall, bone marrow, food, feed, and mixtures, dilutions, or extracts thereof.

The sample may also relate to assessment of particles in water, such as control of drinking water, control of waste water or water from a water purifying plant or swimming pool. In all applications the control may be related to the total particle count, such as bacteria count or it may more particularly be related to a monitoring process for specific bacteria, such as pathological bacteria.

Furthermore, fermentation control, i.e. control of cell growth and viable cells in fermentation tanks may be conducted by the invention. This relates to all technical and industrial fields using fermentation, such as the pharmaceutical industry for producing peptide or protein composition.

The liquid sample may be pre-treated with any suitable treatment, such as centrifugation, sedimentation, filtration, extraction, dilution, irradiation, agitation, addition of chemicals, chromatographic separation.

In another embodiment the sample is a solid sample which is pre-treated prior to being arranged in the sample compartment. An example of pre-treatment is blending optionally followed by any of the treatment mentioned for the liquid sample.

The sample may be any biological sample, such as a biopsy of tissue, such as a biopsy of muscle, brain, kidney, liver or spleen.

Also, the sample may be a sample of food or feed to be tested for contamination, such as bacterial contamination. The present invention offers a very fast method of detecting and enumerating bacteria in food or feed such as a method of detecting Salmonella species.

Independent of the form of sample it is required that the cell is suspended in a medium before contacting the substrate. Said medium may be the natural medium for the cell or any liquid suitable for the detection. In one embodiment the cell is suspended in a medium after being pre-treated. The medium may comprise the catalyst if appropriate.

### Catalyst

By the term catalyst is meant any compound capable of converting or aiding in the conversion of a substrate into a product being detectable by the detection means. The catalyst may thus be an inorganic catalyst as well as an organic catalyst. In one embodiment the catalyst is an enzyme wherein the term enzyme is used in its normal meaning. Where enzymes are used for labelling either a single enzyme, an oligomeric form of the enzyme, or an enzyme/anti-enzyme complex may be used.

The enzyme may be any enzyme useful in an ELISA technique such as an enzyme selected from the group consisting of phosphatases such as alkaline phosphatase, β-galactosidase, peroxidases such as for example horseradish peroxidase, β-glucuronidase, β-glucose-6-phosphate dehydrogenase, glucose oxidase, urease, luciferase, β-lactamase and β-amylase.

When two, three or more different types of cells are detected in one turn, the different enzymes must be selected so that they do not interfere with one another. For example, enzymes are advantageously chosen that require approximate the same pH. Advantageously, substrates should be selected that are only converted to product by one of the two or more present enzymes. Similarly, the substrates may preferably be chosen so that neither the substrates nor their products inhibit any of the enzymes present in the sample compartment.

Further, the enzyme may be coupled to an alternative detection system, such as an amplification system, such as a avidin-biotin anti-peroxidase technique (ABAP). The label may also be biotin, whereby the biotinylated antibody is detected using a labelled avidin or streptavidin, which is enzymelabelled. In a preferred embodiment the avidin or streptavidin is labelled with one of the enzymes discussed above. In another embodiment the label is avidin or streptavidin whereby the antibody is detected using a biotin, which is enzymelabelled. By labelling with biotin/avidin or streptavidin it is possible to enhance the signal as compared to labelling directly with enzyme and more preferred the enzyme is horse radish peroxidase.

Several embodiments involve the use LNA and PNA when the cell contains DNA or RNA material.

In one embodiment the catalyst reaction may be controlled such as controlled by temperature or illumination or light exposure, so that the kinetics of the catalyst reaction is controlled. For example it may be controlled that the catalyst reaction does not commence before initiated, for example by changing the local temperature of the catalyst, or by exposing the catalyst to light such as illumination to remove a substrate or co-factor, or illumination to convert a compound into a substrate or a co-factor. In another embodiment the catalyst reaction is controlled by pH, so that changes in the pH may increase the catalyst reaction. Also in a preferred embodiment it is possible to stop the reaction catalysed by the catalyst externally such as by controlling a temperature shift or the like.

### Species-specific linkage

The term species-selective linkage is used synonymously with the term species-specific linkage, i.e. a linkage that is specific for the cell the parameter of which is to be assessed. In one embodiment the species-selective linkage is antigen-antibody binding, using antibodies, such as monoclonal antibodies, directed to an epitope on the cell.

The monoclonal antibodies may be labelled directly or indirectly. Direct labels typically include catalysts, such as enzymes, and biotin. Indirect labels include antibodies against the monoclonal antibody, said antibodies being labelled with catalyst labels, such as enzyme labels, or biotin.

In an indirect label the primary antibody or nucleotide probe directed against an epitope or nucleotide sequence on the cell may be linked covalently to a compound such as biotin, streptavidin, avidin, a hapten, digoxigenin, dinitrophenyl or fluorescein. The cell may then be visualised by a second label which specifically binds to the compound linked to the primary antibody or probe. Examples of such indirect labels include but are not limited to: hapten anti-hapten complex; biotin streptavidin complex; biotin avidin complex; digoxigenin anti-digoxigenin complex; dinitrophenyl anti-dinitrophenyl complex; fluorescein anti-fluorescein complex.

In the case where the primary antibody or probe is linked to biotin, signal amplification can be obtained by linking streptavidin to the biotin and further linking biotin-enzyme-biotin complexes to the streptavidin. Further rounds of amplification can be obtained by linking further streptavidin and biotin-enzyme-biotin complexes. The result is that several enzymes are linked via complex linkages to the epitope or nucleotide sequence instead of just one enzyme.

In another embodiment the species-selective linkage may be provided using DNA and/or RNA and/or PNA and/or LNA probes selective for DNA and/or RNA related to the cell. The probes may be labelled directly or indirectly through additional probes as described above in relation to labelling of antibodies.

The invention also comprises the feature that an additional linkage is formed between a second species of cell and a second catalyst.

The linkage may be formed in the sample compartment by arranging the cells and the catalyst in the sample compartment and allowing the cell-catalyst complex to form. In another embodiment the cell-catalyst complex is formed before the sample or cells are transferred to the sample compartment.

After forming the cell-catalyst complex excess catalyst not being linked to the species of cells is preferably removed from the cell-catalyst complexes. The excess catalyst may be removed through any suitable means, such as centrifugation, filtration and/or through flushing.

The steps of removing excess catalyst may also comprise binding the cell-catalyst complex to a magnetic bead.

In other embodiments of the present invention the excess catalyst is substantially not removed from the cell-catalyst complex. This obviously implies a more complex conditions under which any image is to be recorded since signals are generated not only in, at or in the vicinity of the cell but also in the media surrounding the cell. An example of where it could be possible to detect signals originating from products produced in, at or in the vicinity of the cell is where the concentration or efficiency of the catalyst in the cell-catalyst complex is greater than in the media surrounding the cell.

The catalyst-cell complex may further be contacted with co-factors or a buffer before contacting the complex with the substrate.

In the following non-exclusive list of examples of species specific linkages formed between the cell and the catalyst, an asterisk ^{*} denotes an affinity binding such as the binding between antibody and antigen or the binding between avidin and biotin. A dash - denotes a covalent bond.
cell*antibody-catalyst
cell*antibody*antibody-catalyst
cell*antibody-biotin*avidin-catalyst
cell*antibody-biotin*streptavidin-catalyst
cell*antibody-avidin*biotin-catalyst
cell*antibody-streptavidin*biotin-catalyst
cell*antibody*antibody-avidin*biotin-catalyst
cell*antibody*antibody-streptavidin*biotin-catalyst
cell*antibody*antibody-avidin*biotin-catalyst
cell*antibody* antibody-streptavidin*biotin-catalyst
cell*antibody-biotin*streptavid in*(biotin-catalyst-biotin)ₙ*streptavidinₙ etc
cell*antibody-digoxigenin*antidigoxigenin-catalyst
cell*antibody-dinitrophenyl*antidinitrophenyl-catalyst
cell*antibody-hapten*antihapten-catalyst
cell*antibody-fluorescein*antifluorescein-catalyst
cell*DNAfragment-catalyst
cell*DNAfragment*DNAfragment-catalyst
cell*RNAfragment-catalyst
cell*RNAfragment*RNAfragment-catalyst
cell*PNAfragment-catalyst
cell*PNAfragment*PNAfragment-catalyst
cell*LNAfragment-catalyst
cell*LNAfragment*LNAfragment-catalyst
cell*DNAfragment*RNAfragment-catalyst
cell*DNAfragment*LNAfragment-catalyst
cell*DNAfragment*PNAfragment-catalyst
cell*RNAfragment*LNAfragment-catalyst
cell*RNAfragment*PNAfragment-catalyst
cell*RNAfragment*LNAfragment-catalyst
cell*nucleotide probe-biotin*streptavidin*(biotin-catalyst-biotin)ₙ*streptavidinₙ etc
cell* nucleotide probe -digoxigenin*antidigoxigenin-catalyst
cell* nucleotide probe -dinitrophenyl*antidinitrophenyl-catalyst
cell* nucleotide probe -hapten*antihapten-catalyst
cell* nucleotide probe -fluorescein*antifluorescein-catalyst

It is likewise conceivable that the species specific linkage may be formed by reversing the position of two components participating in the affinity binding such as reversing the order of avidin*biotin or RNA*PNA etc.

### Substrate

The substrate is typically added to the cell-catalyst complex before recording any images of the sample compartment.

The substrate may be mixed with cell-catalyst complex before arranging the mixture in the sample compartment. In order to avoid substantially any catalyst reaction to take place before the mixture is in place, the catalyst reaction may be controlled as described above in relation to the catalyst.

In another embodiment the cell-catalyst complex is arranged in the sample compartment before adding the substrate, where the mixture of substrate and cell-catalyst is formed in the sample compartment.

### Catalysed reporter deposition

The catalyst cell complex according to the invention may be amplified through the use of catalysed reporter deposition. By catalysed reporter deposition is meant the deposition, such as covalent bonding, onto the cell of a number of reporter molecules. By this deposition of reporter molecules the number of catalysts linked to one species of cell can be increased and thereby the signal originating from a single species of cell can be increased. It is to be understood that reporter molecules encompass any molecule to which a catalyst according to the invention can be linked, e.g. through a species specific linkage.

Typically the reporter molecule comprises two components, a substrate, which will form the linkage to a receptor on the cell, and covalently linked to the substrate component one part of an affinity binding pair.

In the following the principle of reporter activated deposition is explained using tyramin-biotin as an example. It is to be understood that tyramin-biotin is used for illustrative purposes only, and that other examples of reporters that can be used in catalysed reporter deposition are available to the skilled practitioner.

The first step in the procedure involves the binding of a peroxidase to the cell through a species specific linkage, e.g. through linkage via a primary antibody directed against an epitope on the surface of the cell.

After binding of the primary antibody, tyramin-biotin and H₂O₂ is added. Through oxidation by the peroxidase, the tyramin component of the reporter molecule is activated and forms a covalent linkage to electron rich moieties, such as to tyrosin or tryptophan residues, on the surface of the cell within close distance of the peroxidase. The end result is that a number of biotin molecules are bound covalently to the cell to which the peroxidase was first bound. In a second round of labelling, a further catalyst linked to avidin or streptavidin is added to the cell to form linkages between the biotin linked to the cell. The end result is thus that numerous catalysts are linked to each cell for every primary antibody linked to the cell initially.

A number of examples of the substrate component of the reporter molecule are disclosed in US 5,196,306.

### Chromography/luminescence

The substrate may be a chromogenic substrate or a luminogenic substrate, for example a photoluminogenic, an autoluminogenic or chemoluminogenic substrate such as a fluorogenic substrate.

A chromogenic substrate leads to a coloured product being detectable by measuring the absorbance. Any suitable chromogenic substrate used for conventional ELISA may be used herein, as long as the colour of the spot is distinguishable from the background.

A luminogenic substrate leads to a product capable of emitting photons, either when excited or by itself.

Examples of substrate systems which can be used in many embodiments of the present invention are:
AttoPhos (TM associated to JBL Scientific Inc. (San Louis Obispo, CA). The chemical formula of AttoPhos reported differently by the two references: Zahra P. et al.; In Vitro Cell. Biol. - Animal 34: 772-776, 1998; A fluorometric assay for the measurement of endothelial cell density in vitro: 2'-[2-benzthiazoyl]-6'-hydroxy-benthiazole phosphate, or Yu H. et al.; Analytical Biochemistry 261: 1-7, 1998; Development of a magnetic microplate chemifluorimmunoassay for rapid detection of bacteria and toxin in blood: (2'-[2-benzthiazoyl]-6'-hydroxy-benzthiazole phosphate bis-[2-amino-2-methyl-1, 3-propanediol])
4-MUP (4-methylumbelliferyl phosphate)
HNPP ( Roche no. 1 758 888)
4-MUG (4-methylumbelliferyl beta-galactoside)
CDP-Star (Roche no. 1 685 627)
CSPD (Roche no. 1 655 884)
Super Signal Substrate (Pierce, Rockford, III). No chemical formula given. Reference: Trevanich S et al., Journal of Food Protection 63: 534-538, 2000; Rapid Detection of Enterotoxigenic Escherichia coli 06 in Water by Using Monoclonal Antibody and Photon-Counting Television Camera.
Luminol/4-iodophenol (e.g. BM Chemiluminescence ELISA Substrate (POD) - Roche no. 1 582 950).
Galacton Plus with and without enhancer (e.g. BM Chemiluminescence ELISA Substrate - Roche no. 1 759 787).
DAB (3,3'-diaminobenzidine tetrahydrochloride) with and without metal enhancer
OPD (o-phenylenediamine dihydrochloride) or free base
AEC (3-amino-9-ethylcarbazole)
5AS (5-aminosalicyclic acid)
2,2'-azino-bis (3-ethylbenzthiazoline-6-sulfonic acid)
4C1 N (4-chloro-1-naphtol)
o-dianisidine (3,3'-dimethoxybenzidine)
TMB (3,3',5,5'-tetramethylbenzidine) free base or dihydrochloride
ABTS
BCIP (5-bromo-4-chloro-3-indoyl phosphate) with and without NBT (nitro blue tetrazolium) or 2-(iodophenyl)-5-(4-nitro-phenyl)-3-phenyltetrazolium chloride)
Fast Red/Naphthol AS-MX (Sigma F4648)
Naphthol AS-TR phosphat (Sigma N8518) with an dwithout Fast Red RC (Sigma F5146)
pNPP (p-nitrophenyl phosphate)
PMP (phenolphthalein monophosphate - Sigma A3344)
X-Gal (5-bromo-4-chloro-3-indoyl-beta-D-galactopyranoside
CPRG (Chlorophenol-beta-D-galactopyranoside) EP patent 0146866 owned by Roche Diagnostics GmbH)

### Chromogenic substrates

According to a preferred embodiment, the substrate is a chromogenic substrate, which can be detected by measuring it's absorbance. According to an especially preferred embodiment, the coloured product formed from the chromogenic substrate precipitates upon formation. In this way, it is ensured that the detectable product remains in the vicinity of the cell to be detected. When the sample compartment is kept horizontal during detection, the product formed will simply precipitate on the lower surface of the sample compartment and form an insoluble precipitate there. Substrates that are especially suitable for this type of detection are those with a low water solubility.

In some cases it may be advantageous to add a filter to the detection device. Preferably the filter should be selective for the product to be determined. The advantage of the filters is that they can effectively filter away any background signals not coming from the relevant product. The system may also be equipped with several filters for the detection of two, three or more differently coloured products.

If the detector is a colour sensitive CCD there may be no need for colour specific filters.

### Fluorescence

In a preferred embodiment the substrate is a fluorogenic capable of being converted into a fluorescent product by the catalyst. A system based on fluorescence is generally more sensitive than a chromogenic since fewer product molecules are necessary for recording an image from the sample compartment. Therefore a shorter incubation time is generally necessary for a sufficient amount of product molecules to be produced and the image may be recorded faster than when using a chromogenic substrate.

A fluorogenic substrate preferably leads to a fluorescent product emitting signals in the wave length range of from 300 to 1200 nm when excited by excitation light. One preferred fluorescence method is the method of polarised fluorescence.

The excitation light source is any suitable light source capable of emitting excitation light in the range of from 250 nm to 600 nm, such as a light emitting diode (LED), a gas laser, a solid state laser, a laser diode, a gas lamp, a halogen lamp, or a xenon lamp.

It is preferred to use a diverging excitation light, such as light emitting diodes for in a cost-effective manner to expose as large area as possible of the sample to the excitation light.

It may be preferred to use more than one light source for the purpose of increasing the flux of light onto the sample, for instance by using two or more light emitting diodes. It is also possible to use more than one light source where some of the light sources have different electromagnetic properties.

By the use of several LEDs the sample is exposed to excitation light from several angles leading to a substantially optimal excitation of the sample, the light source are preferably operated in such a way that all transmit substantially simultaneously.

However for some application wherein at least a first and a second light sources are arranged in the first excitation light means, the first light source having a different wavelength band than the second light source, the light sources may transmit in an alternating manner. By the use of two different light sources it is possible to obtain two different fluorescence signals from the sample. There is no upper limit to the number of LEDs used, but often as many as 30 LEDs are provided, such as up to 20 LEDs.

If a less diverging light source is used a diverging optical means may be arranged in the excitation light path to diverge the excitation light properly.

When using laser diodes as the excitation light the proper divergence may be accomplished by an arrangement of at least 4 laser diodes optionally provided with diverging means.

The incident angle of the excitation light is preferably in the range between 30° and 90°, more preferably between 45° and 85°, such as between 50° and 85° to provide a suitable excitation of the sample.

The excitation light may be transmitted directly to the sample, i.e. without being deflected by a beam splitter or the like whereby it is possible to construct the system and apparatus more compact.

### Dual or multiple colour

In a preferred embodiment the correlation comprises distinction between at least two spectral properties of product. Thus, by the present invention it is possible to simultaneously detect at least two different types of cells. This is achieved by using at least two antibodies directed towards two different cells, and providing the two antibodies with two different enzymes, either directly or indirectly, and then further providing the relevant substrates for simultaneous or substantially simultaneous detection of the two different cells due to evaluation of two different spots.

The spots arising from the cells may either have two different colours, or one may be coloured and the other fluorescent, or both may be fluorescent emitting in two distinct wave length areas.

The two different types of cells may be two different cells, for example specific IgG and IgA-secreting cells, or the two different states of the same cell, such as to distinguish between dead and living cells.

Also, in the latter situation the a dual labelling may be carried out by using one labelled antibody towards the cell and then another type of labelling of the cell to distinguish dead from living cells, such as conventional vitality staining.

It is understood, that more than two different cells may be assessed hereby, such as three cells for example.

### Sample compartment

The sample compartment established according to the present invention may be a compartment or an equivalent thereof, wherein the sample is located during recording, such as a three-dimensional sample compartment.

The sample compartment may be a part of a flow-through system, wherein each sample is part of a series of samples, whereby one sample is replacing the previous sample in the sample compartment.

In another embodiment the sample compartment is part of a cassette, such as a disposable cassette as described in WO 00/28297.

The sample is contained in the interior of the sample compartment, which normally has an average thickness of between 20 µm and 2000 µm, usually between 20 µm and 1000 µm and in many practical embodiments between 20 µm and 200 µm.

Normally, the sample compartment has dimensions, in a direction substantially parallel to a wall of an exposing window, in the range between 1 mm by 1 mm and 10 mm by 10 mm, but it will be understood that depending on the design, it may also be larger and, in some cases, smaller.

The part of the sample compartment allowing signals to be detected is referred to as the exposing window that can be as little as 0.01 mm² or more, preferably with an area of 0.1 mm² or more, more preferably with an area of 1 mm² or more, preferably with an area of 2 mm² or more, preferably with an area of 4 mm² or more, preferably with an area of 10 mm² or more, preferably with an area of 20 mm² or more, preferably with an area of 40 mm² or more, more preferably with an area of 100 mm² or more, preferably with an area of 200 mm² or more, preferably with an area of 400 mm² or more, preferably with an area of 1000 mm² or more, preferably with an area of 2000 mm² or more, preferably with an area of 4000 mm² or more, preferably with an area of 10000 mm² or more. Similarly, it is advantageous to extend the window of the sample compartment in a direction which is parallel to the plane of any window exposing signals from the sample to the exterior in order to extend the area of the exposing window and thus increase the volume of the sample which is exposed to the exterior.

Concerning the spatial definition of the shape and size of the area of an sample compartment or a window exposing signals to the detection device there are at least two feasible methods for substantially reliable definition of the size and shape of this area. The first, and in many embodiments preferred method, is to adapt the detection device to be sensitive to exposed signals from a substantially defined area of the exposing window, e.g. by adapting any focusing means of the detection device. The second method, which is in particular preferred when it is difficult to adapt the sensing area of the detection device, is to define the boundaries of such exposing area of the sample compartment, e.g. either by controlling the dimensions of the sample compartment which define the exposing area, or by forming a mask or and effective window defining the exposing area, either in or on the disposable device or in connection with the detection device.

The requirements of the wall of the sample compartment are in particular that the wall allows the signals to pass without any significant limitations. In practice no upper limit is given for the wall thickness apart from what is defined by cost and design. The wall is preferably a substantially stable wall, which leads to a lower thickness limit for each material used. Preferably, the wall is from 0.1 mm to 2 mm, such as from 0.5 mm to 1.5 mm, more preferred from 0.75 mm to 1.25 mm.

In some embodiments, a flexible wall is useful, however, for quantitative measurements this will require measurement of the volume of the sample exposed before the assessment is carried out.

### Sample volume

Sample volumes as small as 1 ml or less and even as small as 0.02 ml can be used. The optimal volume of the sample needed is highly dependent on the number of cells present in the sample and the predetermined statistical quality parameter sought.

Other preferred embodiments of the present invention make it possible to assess cells from a considerably large volumes of sample. This can allow the measurement of samples with only few cells of interest per volume of sample. Sample volumes larger than 1 ml and even larger than 100 ml can be used for the analysis, the volume being defined as the total volume of any liquid sample introduced to any flow system connected to the device before the measurement of the sample.

Often the design of the sample compartment or the sample is such that the size of the volume of the liquid sample is sufficiently large to permit the assessment of the at least one quantity parameter or the at least one quality parameter to fulfil a predetermined requirement to the statistical quality of the assessment based on substantially one exposure, so that the image is recorded in one exposure. In another embodiment the assessment of at least one quality parameter or at least one quantity parameter is done by correlating more than one image to the at least one quality parameter or at least one quantity parameter, preferably by correlating two images, more preferably correlating more than two images, more preferably correlating more than four images. In these situations the images are recorded through two, three or more exposures.

Also, information about the changes in the image in course of time is used in the assessment of at least one quality parameter or at least one quantity parameter, and in such situations more than one exposure is made.

In many assessments of cells it is of interest to allow exposure of signals from substantially large volumes of sample. The volume of the liquid sample from which signals such as electromagnetic radiation is exposed onto the detection system is normally in the range between 0.01 µl and 20 µl. Generally the volume of the sample being analysed should be as large as possible. This allows the simultaneous assessment of a higher number of cells, but the optimal volume is often defined by one or more aspects of the detection system and the sample being analysed. Thus the volume of the sample in the sample compartment can be less than 0.1 µl but often volume of more then 0.1 µl, 1.0 µl or even 10 µl is used. In still other application volume of the sample compartment as large as 100 µl or more can be used.

A large volume of the sample is preferably measured by passing the volume of sample through a cell retaining means, such as a filter, electrical field, magnetic field, gravitational field, such means preferably being included in the device or can be arranged to interact with any sample within the device. The cell retaining means should preferably be able to retain substantially all cells present in a sample, or at least a substantially representative fraction of at least one type of cell present in the sample.

When the cells from a large sample are retained, those cells can be resuspended in a volume which is less than the volume of sample passed through the cell retaining means.

In one embodiment more than one portion of the same sample material can be subjected to analysis by exposure to the detection system. This can be done by allowing the sample compartment to be moved, thus exposing a different portion of the sample compartment.

### Magnification

The method is preferably carried out at a low magnification whereby it is possible to detect spots in a large volume in one or a few exposures. The magnification factor is preferably below 20, such as below 10, such as below 5, such as 4, more preferably below 4, such as 2, more prefereably below 2, such as 1. The advantage of such low magnification are several, among other things increased area of observation and increased depth of focusing implying increased volume exposed to the detection device.

When the spots in question have dimensions which are comparable to the size of a detection element, it is often preferred to have magnification of about 1/1, thus focusing the image of any spot on any one or just few detection elements. This can under some condition give favourable detection of any signal.

When analysing spots which have dimensions which are comparable to, or bigger than the detection elements used, it is often advantageous to reduce the size of the image of such spot, to a degree where the size of the image is comparable to the size of a detection element. Thus in one embodiment it is preferred that the magnification factor below 1, preferably below 0.9, such as 0.8, more preferably below 0.8 such as 0.6, more preferably below 0.6 such as 0.5.

In these situations it is preferred that the ratio of the size of a spot, to the size of the image of the particle on the array of detection elements is 1/1 or less, preferably less than 1/1 and higher than 1/100, more preferably less than 1/1 and higher than 1/40, more preferably less than 1/1 and higher than 1/10, more preferably less than 1/1 and higher than 1/4, more preferably less than 1/1 and higher than 1/2.

Thus, it is often preferred that the spatial representation exposed onto the array of detection elements is subject to such a linear enlargement that the ratio of the image of a linear dimension on the array of detection elements to the original linear dimension in the sample compartment is smaller than 40:1, normally at the most 20:1, preferably smaller than 10:1 and in many cases even at the most 6:1 or even smaller than 4:1.

The aspect ratio of an image can be considerably distorted on the array of detection elements, without that having considerable negative effect on the assessment of cells. In such a situation it preferred that the ratio of the shorter to the longer of the two dimensions of the image of a particle on the array of detection elements is substantially 1 or less, preferably 1/2 or less, more preferably 1/4 or less, more preferably 1/10 or less, more preferably 1/50 or less, more preferably 1/100 or less, more preferably 1/200 or less, relative to the ratio of the corresponding dimensions of the particle. In such situation the ratio of the shorter to the longer of the two dimensions of the image of a particle on the array of detection elements is in certain circumstances substantially not the same within the area spanned by the array of detection elements.

It is often preferred that the image of the product from the individual cells the parameter or parameters of which is/are to be assessed are imaged on at the most 25 detection elements, in particular on at the most 16 detection elements and more preferred at the most 9 detection elements. It is even more preferred that the image of the product from the individual cells the parameter or parameters of which is/are to be assessed are imaged on at the most 5 detection elements, or even on at the most 1 detection element. The larger number of elements per cell will provide more information on the individual cells, while the smaller number of elements per cell will increase the total count that can be made in an exposure.

### Statistics

As mentioned above, the size of the volume is suitably adapted to the desired statistical quality of the determination. Thus, where the determination is the determination of the number of cells in a volume, or the determination of the size and/or shape of cells, the size of the volume of the liquid sample is preferably sufficiently large to allow identification therein of at least two of the cells. More preferably, the size of the volume of the liquid sample is sufficiently large to allow identification therein of at least four of the cells. This will correspond to a repeatability error of approximately 50%. Still more preferably, the size of the volume of the liquid sample is sufficiently large to allow identification therein of at least 10 of the cells. This will correspond to a repeatability error of approximately 33%. Even more preferably, the size of the volume of the liquid sample is sufficiently large to allow identification therein of at least 50 of the cells. This will correspond to a repeatability error of approximately 14%. Evidently, where possible, it is preferred to aim at conditions where the size of the volume allows identification of even higher numbers. Thus, when the size of the volume of the liquid sample is sufficiently large to allow identification therein of at least 100 of the cells, it will correspond to a repeatability error of approximately 10%, and when the size of the volume of the liquid sample is sufficiently large to allow identification therein of at least 1000 of the cells, it will correspond to a repeatability error of as low as approximately 3%.

### Stand still

In a preferred embodiment of the invention the cells being assessed are substantially at stand-still during analysis, thus allowing the optimal use of measurement time in order to improve any signal to noise conditions. This arrangement also eliminates any error which could be inherent in the assessment of cells caused by variation in flow conditions, particularly when an assessment of a property is a volume related property such as the counting of cells in a volume of sample.

### Flow system

The introduction of cell, catalyst and substrate into the sample compartment may be provided by means of a flow system. The flow system may provide at least one of several operations to be carried out on the samples, said operations being selected from but not limited to transport, mixing with reagent, homogenising of sample and optionally reagent, heat treatment, cooling, sound treatment, ultra sound treatment, light treatment and filtering.

In order to flow the sample into or within or out of the sample compartment it is preferred to have at least one propelling means provided in the system.

Preferably the flow regulation means is arranged to function stepwise so that the sample and/or the reagent component may be flowed stepwise through the system.

The sample in the device can be flown by the means of a flow system, which can be driven by a pump or a pressurised gas, preferably air, or by causing a pressure difference such that the pressure on the exterior of the inlet is higher than the pressure within at least a part of the system thus forcing the sample to flow through the inlet. In many embodiments of the present invention the flow in said flow system is controlled by one or more valves which can adjust the flow speed of the sample. In many preferred situations the flow of liquid in the device can be brought about by a vacuum, the vacuum being applied from a reservoir, preferably contained within the device. The vacuum can be established by a mechanical or physical action creating the vacuum substantially simultaneously with the introduction or the movement of the sample. These mechanical or physical actions can be: a peristaltic pump, a piston pump, a membrane pump, a centrifugal pump and a hypodermic syringe.

The outlet from the sample compartment can be passed through a flow controlling means, such as a valve, which only allows gas to pass through. One such type of valves which often is preferred, is one which allows gas and air to pass but can close irreversibly when the valve comes in contact with liquid sample. The effect of such valve is to minimise the movement of any sample within the sample compartment during analysis.

In a preferred embodiment of the invention the system contains at least one compartment wherein the mixing of the sample material with catalyst and/or media is possible.

One advantage of the present system and method is that the analysis is carried out using only liquid reagents and cells suspended or dissolved in liquid. This layout ensures ease of operation and handling. Surprisingly it has been determined that it is possible to detect cells specifically in the absence of bonds to any solid support.

### Detection device

The image which can be detected from the window of the device can for instance be detected by an array of detection elements, the array of detection elements comprising individual elements each of which is capable of sensing signals from a part of the sample window area, the array as a whole being capable of sensing signals from substantially all of the sample window area, or at least a well defined part of the sample window area. The array of detection devices may for example be a one-dimensional array or a two-dimensional array. In order to facilitate the assessment of cells the intensities detected by the array of detection elements are processed in such a manner that representations of electromagnetic signals from the cells are identified as distinct from representations of electromagnetic background signals.

The detection means may comprise any detectors capable of sensing or detecting the signal emitted from the sample such as a fluorescence signal.

In a preferred embodiment detection means comprises a detector being an array of detecting devices or detection elements, such as a charge coupled device (CCD) the CCD may be a full frame CCD, frame transfer CCD, interline transfer CCD, line scan CCD, an eg. wavelength intensified CCD array, a focal plane array, a photodiode array or a photodetector array, such as a CMOS. The CMOS is preferably a CMOS image sensor with on-chip integrated signal condition and/or signal processing. Independent of the choice of any of the above detection devices the detection means may further comprise a white/black or colour CCD or CMOS.

Confocal scanning optical microscopes are known in the art and offer a number of advantages over traditional optical microscopes. One main advantage of a confocal scanning microscope is that it provides optical sectioning of a sample because it attenuates light which is not in focus. Thus, only light which is in focus contributes to the final image.

In a scanning confocal microscope, a beam is swept across a surface of a sample. The light which emanates from the sample (e.g., reflected from, emitted from or transmitted through) is directed towards a pinhole. Light that is in focus passes through the pinhole and onto an optical detector. As the beam is scanned across the surface of the sample, the output from the optical detector can be accumulated and formed into an image of the scanned surface.

Use of a confocal scanning microscope especially a confocal laser scanning microscope for detecting the signals from the product formed in the sample compartment is advantageous due to the greater sharpness of the detected image.

The size of the detection elements determines to some extend its sensitivity. In some applications it is therefore of interest to have detection elements of size of about 1 µm² or less. In certain situations the size of the detection elements in the array of detection elements is less than 20 µm², preferably less than 10 µm², more preferably less than 5 µm², more preferably less than 2 µm², more preferably less than or equal to 1 µm². In other situations the size of the detection elements in the array of detection elements is greater than or equal to 5000 µm², such as greater than or equal to 2000 µm², more preferably greater than or equal to 1000 µm², such as greater than or equal to 500 µm², or even greater than or equal to 200 µm², more preferably greater than or equal to 100 and less than 200 µm², more preferably greater than or equal to 50 and less than 100 µm², more preferably greater than or equal to 20 and less than 50 µm².

The array of detection elements is preferably sensitive to electromagnetic radiation of wavelength in one or several of the following regions: 100 nm to 200 nm, 200 nm to 600 nm, 300 nm to 700 nm, 400 nm to 800 nm, 600 nm to 1 µm, 800 nm to 2 µm, 2 µm to 10 µm, 5 µm to 10 µm, 10 µm to 20 µm, 20 µm to 40 µm.

The inclusion of a focusing device for the focusing of a signal from the sample onto the detection elements in such a manner as to maximise the collection angle, the collection angle being defined as the full plane angle within which a signal is detected, has in many situations been found to give improved conditions for an assessment. Surprisingly it was found that such a wide collection angle, even to the extent that the objective used in the focusing distorted the aspect ratio of the image of any cell differently across the plane in which the detection elements were placed, or produced variation in the focusing across the sample being analysed, or reduction of the focusing quality, could be used in the assessment of for example the number of cells in the sample.

The aspect ratio of the detection elements can be important in the collection of signals for the assessment of cells. A ratio of about 1/1 is some times preferred, but under some conditions it can be preferred to use ratio different from 1/1. In particular when this facilitates detection of signals from increased volume of any sample, thus allowing simultaneous assessment of for examples more cells. In those circumstances the ratio of the shorter of the height or the width, to the longer of the height or the width of the detection elements in the array of detection elements is substantially equal or less than 1, preferably less than 1/2, more preferably less than 1/4, more preferably less than 1/10, more preferably less than 1/50, more preferably less than 1/100, more preferably less than 1/200.

### Focusing - Lenses

Signals from at least a portion of the sample are focused onto the array of detection elements, by the use of a focusing means, preferably by the use of one lens, it is however possible to use two lenses, or more than two lenses. The number of lenses used for the focusing system can affect the complexity of any measuring system.

The focusing of a signal from the sample onto any detector is dependent on the position of the sample relative to any detector. When the construction of measuring system is such, that the relative position of the sample and any detector can vary, then there is advantage in being able to adjust the focusing of the system. This can often be achieved by first taking at least one measurement of any signal from the sample and then on the bases of this, to adjust the focusing of the system. This procedure can be repeated a number of times in order to obtain acceptable focusing. In the same manner the focusing of signal from the sample or sample material is adjusted, preferably where the extend of the adjustment is determined by at least one measurement of a signal from the sample.

The collection angle of a focusing arrangement used can have effect on the intensity of any signal collected on the array of detection elements. When high sensitivity is needed it is therefore practical to increase the collection angle. The preferred size of the collection angle can also be determined by other requirements which are made to the system, such as focusing depth. In these situations the collection angle of the focusing means is preferably at least 2 degrees, preferably more than 5 degrees, more preferably more then 15 degrees, more preferably more than 20 degrees, more preferably more than 50 degrees, more preferably more than 120 degrees, more preferably more than 150 degrees.

### Signal

The signals measured from one or more detection elements may be corrected for systematic or varying bias by the use of a calculating means, the bias correction being accomplished by the use of one or more pre-defined value(s), preferably where each measured signal for one or more detection elements in said array of detection elements has one or more pre-defined value(s), more preferably where each pre-defined value is determined on the bases of one or more of any previous measurements.

The bias correction may be performed by subtracting the results obtained in one or several of other measurements from the measured signal, preferably where the other measurements are one or several of measurements of the same sample, or sample material, more preferably where the other measurement is the measurement taken previously of the same sample or sample material.

Also the signal from one or more detection elements may be corrected for intensity by the use of a calculating means, said correction being accomplished by the use of one or more pre-defined value(s), preferably where each measured signal for one or more detection elements in said array of detection elements has one or more pre-defined value(s), more preferably where each pre-defined value is determined on the bases of one or more of any previous measurements.

In some situations e.g. in an analogue-to-digital conversion it could also be of interest to adjust the level of 2, preferably 3, more preferably 4, more preferably 5, more preferably 6, more preferably 7, more preferably 8, more preferably more than 8, separate output channels in such a way that one, preferably more than one, of the output channels has/have substantially different level from the other output channel(s), where the identification of which of the output channels, or combination thereof, has substantially different output level, is correlated to the intensity of said signal.

For the analysis of any measured signal it is often necessary to digitalise the signal, in such a way that a given intensity of any signal is transformed into a digital representation. This can be done by having a series of channels, were the information about which of these channels has signal which differs from the other channels determines the intensity, or even by having more than one of this channels forming a combination, preferably in a way similar to binary representation.

### Processor

Information of the signals detected by the detection means are input into a processor for processing, displaying and optionally storing the information.

The signal information may be displayed on a display connected to the processor and/or printed. The information displayed may be any kind of information relating to the signals measured and/or the system used, such as a number, size distribution, morphology, classification of cells, excitation wavelength, emission wavelength, magnification. In particular the data processing means is capable of distinguishing partially overlapping areas of product.

Storage capacity, for instance used for storing information about measured signals from the detection elements, is often one of those components which have considerable effect on the cost of production. It is therefore of interest to be able to perform the assessment of parameters without substantial any use of such storage capacity, such that the assessment of cells in a sample is performed without the use of substantially any storage capacity means being used to store measured signals from the detection elements in the array of detection elements.

On the other hand, it is often difficult to accomplish assessment without the use of any storage capacity, but preferably the amount of such storage capacity should not be more than what is needed to store the information from all measured detection elements, preferably where only a fraction of the information can be stored.

In some situations measured signal from the detection elements in the array of detection elements is stored by means of storage capacity, the storage capacity being able to store a number of measurements equivalent to, or less than, the number of detection elements, preferably less than 1/2 the number of detection elements, more preferably less than 1/4 the number of detection elements, more preferably less than 1/8 the number of detection elements, more preferably less than 1/16 the number of detection elements, more preferably less than 1/32 the number of detection elements, more preferably less than 1/64 the number of detection elements, more preferably less than 1/128 the number of detection elements, more preferably less than 1/256 the number of detection elements, more preferably less than 1/512 the number of detection elements, more preferably less than 1/1024 the number of detection elements in the array of detection elements.

In other certain circumstances it is advantageous that the measured signal from the detection elements in the array of detection elements is stored by means of storage capacity, the storage capacity being able to store a number of measurements greater than the number of detection elements, preferably equivalent to, or greater than, 2 times the number of detection elements, more preferably equivalent to, or greater than, 4 times the number of detection elements, more preferably equivalent to, or greater than, 8 times the number of detection elements, more preferably equivalent to, or greater than, 16 times the number of detection elements, more preferably equivalent to, or greater than, 32 times the number of detection elements, more preferably equivalent to, or greater than, 64 times the number of detection elements, more preferably equivalent to, or greater than, 128 times the number of detection elements, more preferably equivalent to, or greater than, 256 times the number of detection elements, more preferably equivalent to, or greater than, 512 times the number of detection elements, more preferably equivalent to, or greater than, 1024 times the number of detection elements in the array of detection elements.

Other, more complicated aspects of the assessment of parameters, can require the use of considerable amount of storage capacity. In this aspect it can therefore be necessary to have storage capacity which can store more information than is collected in one measurement of the detection elements used.

It is possible to make the correlation and the assessment of the parameters of the sample by using a calculation mean, preferably a digital computer, one commercially available from Analogue Devices (ADSP 2101), equipped with storage capacity which can only store information in amount substantially equivalent to a small fraction of the total number of detection elements, the assessment of the number of objects then being based on substantially real time processing of data, preferably in such a way that the measured information from each detection element, or a line of detection elements, or two or more lines of detection elements, is used for the assessment, substantially without any delay, such as a delay which would otherwise be caused by storing the measured information.

However, it is often preferred to store substantially all measured information by the use of a first calculation mean, preferably a digital computer, before the processing of the information by a second calculation mean, preferably a digital computer, and thus allowing the measured information to be processed at substantially the same rate it is obtained, but with a substantial time delay between the measurement of any information and the processing of the same information; preferably, this is accomplished by using only one calculating mean, preferably a digital computer, equipped with enough resources to accomplish the task.

### Medical markers

The system and the method of the current invention may be used for detection of clinical markers of conditions in a human being or in an animal. One preferred use of the method and system is for the early diagnosis of myocardial infarction or cardial infarct. When cells in the heart suffer or die they leak a number of enzymes into the blood. The blood level of these enzymes raise above the normal levels hours before the patient show acute symptoms of cardial infarct. A reliable and early diagnosis can thus be made by measuring the blood level of cardial related enzymes.

According to prior art techniques the enzymes are measure by subjecting a blood sample to traditional ELISA in the laboratory. The duration of this ELISA detection is several hours. During this period the patient does not show any acute symptoms of cardial infarct. At the moment the diagnosis is ready, the cardial infarct may have developed to a level, where it is too late to save the patient's life.

It is envisaged that the blood levels of these particular enzymes and of other enzymes being medical or clinical markers can be measured rapidly using the detection technique of the present invention.

The system may be used for detection of clinical or medical markers in essentiallly all animals, particularly in mammals such as human beings, cows, horses, poultry, sheep, goat.

### One-sided and two-sided systems

The detection device may be laid out as a one-sided device, i.e. a device for which the light is directed to the sample from the same side of the sample as the side for which the signals are detected.

The detection device may also be laid out as a one-sided device, in which the excitation light is directed to the sample from the same side of the sample as the side for which the signals emitted from the sample are detected.

By this apparatus a variety of advantages have been achieved as compared to conventional fluorescence microscopes. First of all it is possible to arrange the sample to be assessed directly in the sample plane instead of sliding it into the sample plane between the detector and the excitation light. Furthermore it has become possible to detect surface fluorescence of a sample not being transparent.

It is also possible to increase the intensity of the excitation light without compromising the detectors.

Also samples having a nature whereby it is normally not possible to arrange the sample in a microscope may be assessed by the use of the present system, in that the microscope may be placed directly on the sample whereby the surface of the sample simply constitutes the sample plane.

Finally it is possible to produce a more compact and thereby more easily handled apparatus, in that the excitation light means is arranged on the same side of the sample plane as the detector, thus shortening the axis of the apparatus by at least 25% as compared to conventional apparatuses.

By the present invention it is possible to assess parameters of a sample which has up to now only been reliably assessed by the use of flow cytometric equipment. It is possible to assess parameters of a large sample in one exposure thus reducing the statistical errors normally counted for when assessing large samples by assessing only parts thereof per exposure.

Furthermore, it is possible to obtain more than one fluorescence signal from the sample in one exposure thereby facilitating classification of particles of the sample, due to their different fluorescence signals.

Thus, the one-sided apparatus according to the invention may be constructed in a wide variety of combination, which are all within the scope of this invention. In particular the principal combination discussed below are envisaged.

The apparatus may be constructed as a single fluorescence apparatus wherein the light sources and the excitation light filters are identical.

A multiple fluorescence apparatus, such as an apparatus providing at least two different fluorescence signals, may be provided by at least one of the following:
- A first and a second light source, said light sources emitting light of different wavelengths
- A first and a second filter being different whereby the excitation light of at least two different wavelengths are exposed to the sample
- A first and a second emission filter being different, such as a dual band filter, whereby at least two different fluroescence signals are emitted to the detector(s)

It is however a further advantage that the present apparatus may be constructed as a double-sided apparatus, whereby excitation light may be directed onto the sample from both sides of the samples, or detection means are arranged to detect signals from both sides of the samples, or a combination of both.

Thus by a double-sided apparatus is meant an apparatus according to the invention further provided with:
- A second excitation light means located in a second light plane, said second light plane being parallel with the sample plane and located on the other side of the sample plane as opposed to the first light plane. Thereby the sample is receiving excitation light from both sides of the sample considerably increasing the energy exposed to the sample, and/or
- A second detection means arranged so that the sample is positioned between the first detection means and the second detection means. Hereby it is possible to assess different information regarding the signals from the sample by one exposure detection. For example the first detection means may be adapted to register the number of particles of the sample, whereas the second detection means is adapted to register the morphology of the particles in the sample.

In a preferred embodiment the double-sided apparatus comprises both double-sided excitation system and double-sided detection system.

The second excitation light means may be any of the light means discussed in relation to the first light means. Depending on the purpose of the fluorescence microscope the light means may be different or identical.

Furthermore, it may be of interest that the excitation light would constitute different wavelength bands whereby illumination with different wavelengths is achieved.

The second detection means may be any of the detection means discussed in relation to the first detection means.
Any suitable combination of light sources, filters, magnification and detectors are envisaged by the present invention. In the following preferred embodiments of the two-sided system is discussed.

The apparatus may be a single fluorescence system, wherein excitation light of substantially identical wavelength are exposed to the sample from two sides. Thereby the excitation light may be intensified.

In a double-sided excitation light apparatus a first excitation light means exposes the sample to one wavelength from one side of the sample, and the second excitation light exposes the sample to another wavelength from the other side of the sample. It is understood herein, that of course the first excitation light and the second excitation light respectively, may comprise different light source and/or filters, whereby the sample may be illuminated with even more wavelengths as discussed above.

The double-sided excitation light apparatus may comprise one detector, whereby the apparatus functions as a partly transmitting system.

In another embodiment the double-sided excitation light apparatus comprises two detecting means. Thereby an increased amount of information may be obtained from the sample. In one aspect the two detecting means may obtain equal, although mirror images (the images on the two detectors are mirror images of each other), information relating to the sample providing a validation of the information.

The apparatus according to the invention may also be a double-sided detection apparatus using a one-sided excitation light means. Thereby one detector detects signals being transmitted through the sample.

Independent of the arrangement of excitation light, a double-sided detecting system is capable of increasing the amount of information received. For example different wavelength may be received by the two detectors, and or different detectors, having different sensibility may be used. Furthermore, by using for example different magnification for the two detectors the information relating to the sample may be increased. One side of the system may assess for example number of particles in a large area of the sample, for example by a low magnification, and the other side of the system may assess the morphology of the particles by using a larger magnification. Combinations of magnification may for example be 1:1 and 1:4, 1:1 and 1:10, 1:2 and 1:4, 1:2 and 1:10. The signal information transferred from the two detectors is preferably transmitted to the same processor, whereby the information may be displayed separately, as well as being combined providing for example specific morphology information related to specific particles the position and number of which are detected by the other detector.

It is also possible to use the apparatus according to the invention as a double-sided apparatus where the other side is a conventional light microscope or any other type of microscope. When using the other side of the system as a non-fluorescence microscope, the illumination light for the microscope may be suitably arranged on either side of the sample in relation to the microscope.

The double-sided apparatus comprising a conventional microscope on one side, may comprises a one-sided or a double-sided excitation light system for the fluorescence part of the system.

When using a double-sided detection system the processor of the first detection means may receive signal data from the second detection means as well in order to simplify the apparatus. It is however possible to install a separate processor for each detection means.

### Examples of one and double sided excitation and detection systems

In the following one embodiment of the detection system is discussed in more detail in relation to the drawings.

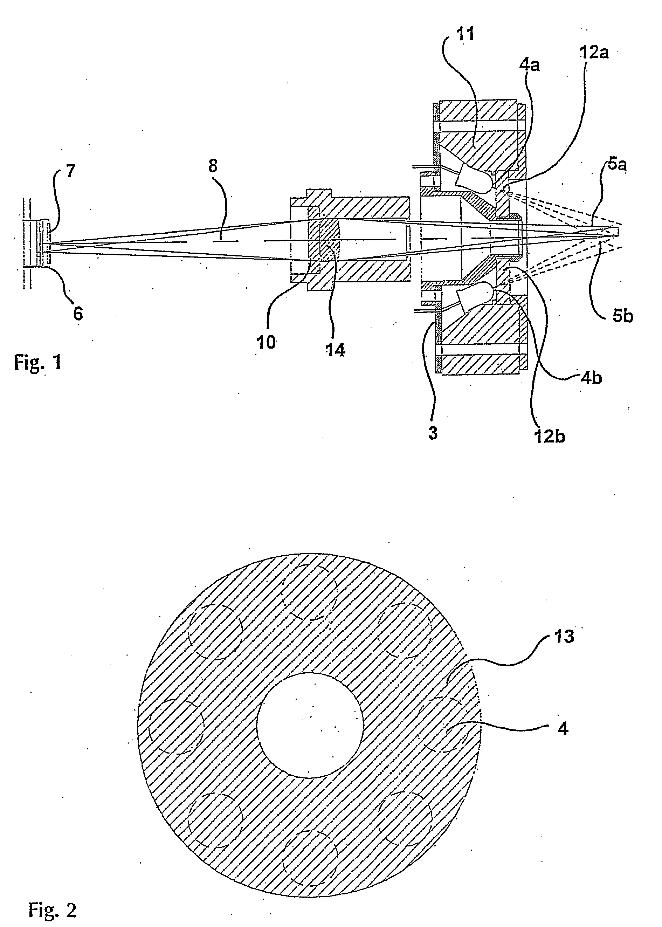

In Fig. 1 an example of the illumination and detection system 1 is shown in schematic form. The sample is arranged in a sample compartment 2 the sample plane. Excitation light from the light sources 4a, 4b in the excitation light means 3 is exposed onto the sample through a main light path 5a, 5b.

Fluorescence signals from the sample is emitted to the detection means 6 comprising at least one detector 7. The path of the emitted signals is following an axis between the sample and the detector, the detection-sample axis 8.

The signal data are transmitted to a processor 9 coupled to the detecting means 6. The fluorescence signals from the sample is filtered by means of emission filter 14 and focused to the detection means 9 by means of a focusing lens 10.

The light sources 4a, 4b are arranged in a light housing 11, whereby the transmission of excitation light directly to the detection means is avoided. Furthermore excitation light filters 12a, 12b are positioned in the excitation light beam.

Fig. 2 shows a cross-section of the circular supporting material 13 of the excitation light filters wherein the position of the light sources have been indicated by circles in broken lines.

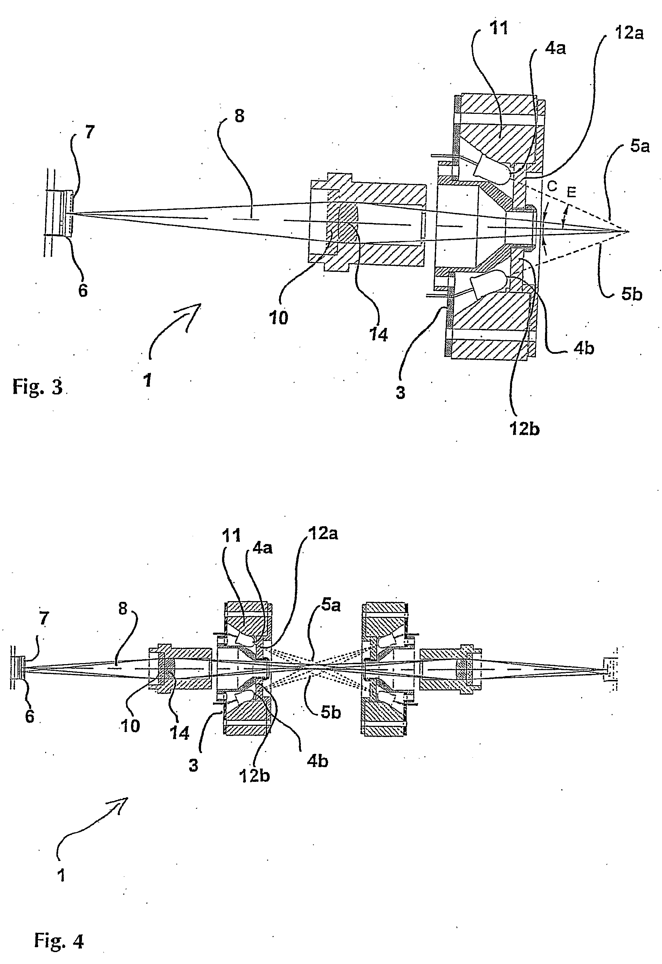

In Fig. 3 the light path and signal path is shown in more detail. In the light path the main light path is shown as 5. Furthermore, the detection-sample axis is shown by broken lines 8. The collection angle of the system is denoted C shown between two arrows and the angle between the main light path and the detection-sample axis is denoted E.

In Fig. 4 a double-sided excitation/detection system 1 is shown wherein the systems on each side of the sample are identical and as described for the one-sided system of Fig. 1.

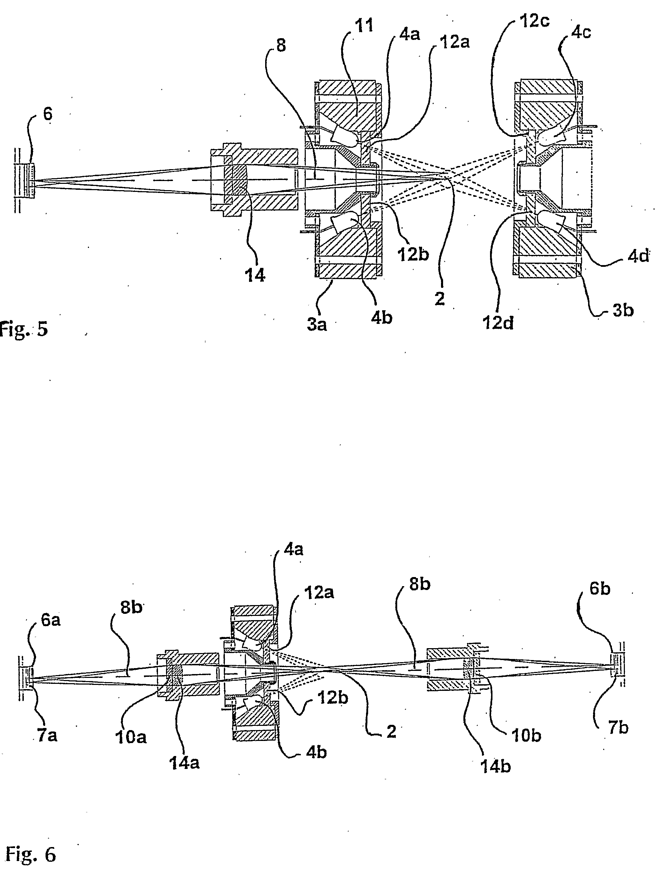

Fig. 5 shows a double-sided excitation system wherein excitation light from the light sources 4a, 4b in the first excitation light means 3a and excitation light from the light sources 4a, 4b in the second excitation light means 3b is exposed onto the sample 2 from both sides of the sample 2. As discussed above, the light sources may be identical or different depending on the information to be assessed. Furthermore, the filters used for each light source may be different or identical.

Fluorescence signals are transmitted through and reflected from the sample due to the excitation light arrangement and emitted to the detection means 6. The path of the emitted signals is following an axis between the sample and the detector, the detection-sample axis 8.

The signal data are transmitted to a processor coupled to the detecting means as described above.

Fig. 6 shows a double-sided detecting system, using a single-sided excitation system, wherein reflected fluorescence signals from the sample 2 are detected by detecting means 6a comprising detector 7a. The reflected fluorescence signals are transmitted though filter 14a and focused by lens 10 a.

Furthermore, transmitted fluorescence signals from the sample 2 are detected by detecting means 6b comprising detector 7b. The reflected fluorescence signals are transmitted though filter 14b and focused by lens 10b.

Filter 14a is preferably different from filter 14b, whereby information relating to at least two different fluorescence signals is obtainable.

Also the magnification in the two detecting systems may be different, for example by lens 10a being different from lens 10b.

### Example

- Sample type: Human whole blood anticoagulated with sodium citrate.
- Cell: Human CD45+ blood cells with CD45 antigen localised on the surface.
- Antibody-conjugate: Monoclonal mouse IgG (Fab) specific for CD45 antigen. IgG is conjugated to biotin (100 µg antibody conjugate per ml of Phosphate Buffered Saline with pH 7.2 (PBS)).
- Avidine-AP conjugate: Streptavidine conjugated to Alkaline Phosphatase (10 µg Avidine conjugate per ml PBS).
- Enzyme substrate: AttoPhos™ Substrate Set (Boehringer Mannheim no. 1681 982). Alkaline Phosphatase will convert the AttoPhos Substrate to a fluorochrome (the Product) which has maximum exitation at 435 nm and maximum emission at 560 nm.
- Lysis Buffer A + B: Uti-Lyse (Dako no. S-3350) is used for lysis of red blood cells but leaving the cell membrane of the white blood cells sufficiently intact in order to carry out a surface marker based cell analysis.
- Microscopic counting: The counting of spots derived from the CD45+ target cells can be carried out using an EPI-fluorescence microscope equipped with a suitable light source (e.g. xenon lamp), a suitable filter set for the Product, a 4x objective, a CCD camera and a Burker-Turk counting chamber (depth 0.1 mm).

A portion of 10 µl of antibody conjugate is added to 100 µl of sample. Following gentle mixing and incubation for 30 minutes at room temperature (RT) a portion of 2 ml PBS is added to the suspension. Following gentle mixing the suspension is centrifuged at 300g for 5 minutes. Then the supernatant is removed and the pellet is resuspended in 2 ml of PBS. Following gentle mixing the suspension is centrifuged at 300g for 5 minutes. Then the supernatant is removed and the pellet is resuspended in 2 ml of PBS. Following gentle mixing the suspension is centrifuged at 300g for 5 minutes. The supernatant is removed (leaving approximately 100 µl fluid) and a portion of 100 µl of Lysis Buffer A is added. Following gentle mixing the suspension is incubated for 10 minutes at RT. A portion of 1 ml of Lysis Buffer B is added to the suspension and incubated for 10 minutes at RT. Then the suspension is centrifuged at 300g for 5 minutes. Then the supernatant is removed and the pellet is resuspended in 2 ml of PBS. Following gentle mixing the suspension is centrifuged at 300g for 5 minutes. Then the supernatant is removed and the pellet is resuspended in 2 ml of PBS. Following gentle mixing the suspension is centrifuged at 300g for 5 minutes.

Then the supernatant is removed and a portion of 100 µl of Avidine-AP conjugate is added to the pellet. Following gentle mixing and incubation for 15 minutes at RT a portion of 2 ml PBS is added to the suspension. Following gentle mixing the suspension is centrifuged at 300g for 5 minutes. Then the supernatant is removed and the pellet is resuspended in 2 ml of PBS and after gentle mixing the suspension is centrifuged at 300g for 5 minutes. After removing the supernatant the pellet is resuspended in 2 ml of PBS and after gentle mixing the suspension is centrifuged at 300g for 5 minutes. After removing the supernatant the pellet is resuspended in 500 µl of AttoPhos™ Substrate solution prepared according to manufacturers instruction. Immediately, a small portion of the suspension is applied to a Burker-Turk counting chamber and then an image is created using the EPI-fluorescence microscope. With short intervals of 1 second further images are created and the formation of dots and the growths of these dots is observed. The dots will increase in diameter due to diffusion of the product.

CD45+ cells can be observed as fluorescent dots within the measuring volume. Based on the number of dots and the volume of the mixture in Burker-Turk counting chamber which is represented by the image a concentration of CD45+ cells in Burker-Turk counting chamber can be calculated. The concentration of CD45+ cells in the blood sample can then be calculated because the dilution factor is known.

## Claims

1. A method for assessing at least one quality parameter or at least one quantity parameter of at least one species of cells in a liquid sample comprising the steps of
establishing a sample compartment having at least one wall,
arranging catalyst-cell complexes between the at least one species of cells and at least one catalyst in the sample compartment in a manner allowing the cells to move relative to the wall(s) of the sample compartment,
arranging a substrate in the sample compartment, said substrate being capable of being converted into a product through catalysing by said catalyst,
contacting the substrate with the catalyst-cell complexes of individual cells allowing a detectable amount of product spots to be produced, the product diffusing from the cells to form localised substantially spherical spots around the cells,
recording an image of the product spots related to individual cells in the sample compartment after a detectable amount of product has been produced and before the various spots become confluent, and
correlating the image to the at least one quality parameter or at least one quantity parameter of the at least one species of cells.

2. The method according to claim 1, wherein the catalyst-cell complex comprises a species-selective linkage.

3. The method according to claim 2, wherein the species-selective linkage comprises an antigen-antibody linkage.

4. The method according to claim 1, whereby the at least one species of cell is a medical marker of a disease.

5. The method according to claim 1, wherein the cells are selected from mammalian cells, insect cells, reptile cells, fish cells, yeast cells, and fungi cells.

6. The method according to claim 1, wherein the cells are selected from blood cells, sperm cells, and bone marrow cells.

7. The method according to any of the preceding claims, whereby the sample is selected from the group consisting of milk, milk products, urine, blood, sperm, nasal secrete, tears, faeces, waste water, process water, drinking water, cerebro-spinal fluid, gall, bone marrow, food, feed, and mixtures, dilutions, or extracts thereof

8. The method according to any of the preceding claims, whereby the substrate is 2-[2-benzthiazoyl]-6'-hydroxy-benthiazole phosphate, 2'-[2-benzthiazoyl]-6'-hydroxy-benzthiazole phosphate bis-[2-amino-2-methyl-1, 3-propanediol], 4-MUP, HNPP, 4-MUG, Luminol/4-iodophenol, DAB, OPD, AEC, 5AS, 2,2'-azino-bis (3-ethylbenzthiazoline-6-sulfonic acid), 4C1N, o-dianisidine, TMB, ABTS, BCIP, Naphthol AS-TR phosphat, pNPP, PMP, X-Gal, or CPRG.

9. The method according to any of the preceding claims, whereby the catalyst is an enzyme.

10. The method according to claim 9, whereby the catalyst is selected from the group consisting of phosphatase, alkaline phosphatase, β-galactosidase, peroxidase, horseradish peroxidase, β-glucuronidase, β-glucose-6-phosphate dehydrogenase, glucose oxidase, urease, luciferase, β-lactamase and β-amylase.

11. The method according to any of the preceding claims, whereby at least one obtained product is photoluminescent or chemiluminescent.

12. The method according to any of the preceding claims, whereby at least one obtained product is fluorescent.

13. The method according to any of the preceding claims, whereby at least one obtained product emits electromagnetic radiation in the range of 300 nm to 1200 nm when exposed to electromagnetic radiation in the range of 250 nm to 600 nm.

14. The method according to any of the preceding claims, wherein at least one product obtained is excited by excitation light prior to recording an image.

15. The method according to claim 3, whereby at least one linkage between the catalyst and the cell comprises two or more antibodies.

16. The method according to claim 3, whereby at least one catalyst is conjugated to an antibody being immunologically bound to an antigen on the species of cell.

17. The method according to claim 16, whereby at least one catalyst is conjugated to a first antibody being immunologically bound to second antibody, being immunologically bound to an antigen on the species of cell.

18. The method according to claim 2, whereby at least one catalyst is conjugated to avidin.

19. The method according to claim 2, whereby at least one catalyst is conjugated to streptavidin.

20. The method according to any of the preceding claims, further comprising the step of removing excess catalyst not being linked to the species of cells.

21. The method according to any of the preceding claims, whereby the step of producing a product is carried out in a liquid environment.

22. The method according to any of the preceding claims, whereby the recording of image comprises the use of a confocal scanner.

23. The method according to any of the preceding claims, whereby the image of product is recorded using an array of detection devices.

24. The method according to claim 23, wherein the image of product is recorded using a two-dimensional array of detection devices.

25. The method according to claim 23, wherein the image of product is recorded using a CCD, a CMOS, a video camera or a photon counting camera.

26. The method according to any of the preceding claims, whereby the image is recorded without magnification.

27. The method according to any of the preceding claims, whereby the image is recorded with a magnification factor below 20.

28. The method according to any of the preceding claims whereby the image is recorded in one exposure.

29. The method according to any of the claims 1-27 whereby the image is recorded in two, three or more exposures.

30. The method according to any of the preceding claims, whereby the recorded image is processed using data processing means.

31. The method according to claim 30, whereby the data processing means distinguish partially overlapping areas of product.

32. The method according to any of the preceding claims, whereby the correlation comprises estimation of the number of spots on the image.

33. The method according to any of the preceding claims, whereby the correlation comprises estimation of the size of spots on the image.

34. The method according to any of the preceding claims, whereby the correlation comprises distinction between at least two spectral properties of product.

35. The method according to any of the preceding claims, whereby the at least one quality parameter is selected from the group consisting of viability, size, identity, respiration, and presence of the cell.

36. The method according to any of the preceding claims, whereby the at least one quantity parameter is selected from the group consisting of number of species of cell in a volume of sample, concentration of species of cell in a volume of sample, amount of species of cell in a volume of sample.

## Patentansprüche

1. Verfahren zum Bewertung wenigstens eines Qualitätsparameters oder wenigstens eines Quantitätsparameters von wenigstens einer Zellenspezies in einer Flüssigprobe, die folgenden Schritte umfassend:
Bilden eines Probenkompartiments, wenigstens eine Wand umfassend,
Anordnen von Katalysator-Zell-Komplexen zwischen der wenigstens einen Zellenspezies und wenigstens einem Katalysator in dem Probenkompartiment, derart, dass sich die Zellen relativ zu der Wand/den Wänden des Probenkompartiments bewegen können,
Anordnen eines Substrats in dem Probenkompartiment, wobei das Substrat durch Katalysieren durch den Katalysator in ein Produkt konvertierbar ist,
Kontaktieren des Substrats mit den Katalysator-Zell-Komplexen einzelner Zellen, wodurch nachweisbare Mengen an Produktpunkten erzeugt werden können, wobei das Produkt von den Zellen diffundiert, um lokal begrenzte, im Wesentlichen sphärische Punkte um die Zellen herum zu bilden,
Aufzeichnen eines Bildes der Produktpunkte, verbunden mit einzelnen Zellen in dem Probenkompartiment, nachdem eine nachweisbare Produktmenge erzeugt wurde, und ehe die unterschiedlichen Punkte konfluent werden, und
Korrelieren des Bildes mit dem wenigstens einen Qualitätsparameter oder wenigstens einen Quantitätsparametern der wenigstens einen Zellenspezies.

2. Verfahren nach Anspruch 1, wobei der Katalysator-Zell-Komplex eine speziesselektive Kopplung umfasst.

3. Verfahren nach Anspruch 2, wobei die speziesselektive Kopplung eine Antigen-Antikörper-Kopplung umfasst.

4. Verfahren nach Anspruch 1, wobei die wenigstens eine Zellenspezies ein medizinischer Marker einer Krankheit ist.

5. Verfahren nach Anspruch 1, wobei die Zellen aus Säugetierzellen, Insektenzellen, Reptilienzellen, Fischzellen, Hefezellen und Funguszellen ausgewählt sind.

6. Verfahren nach Anspruch 1, wobei die Zellen aus Blutzellen, Spermazellen und Knochenmarkzellen ausgewählt sind.

7. Verfahren nach einem der vorhergehenden Ansprüche, wobei die Probe aus der Gruppe ausgewählt ist, die aus Folgenden besteht: Milch, Milchprodukte, Urin, Blut, Sperma, Nasensekret, Tränen, Fäzes, Abwasser, Brauchwasser, Trinkwasser, Zerebrospinalflüssigkeit, Galle, Knochenmark, Essen, Futter, und Mischungen, Verdünnungen oder Extrakten davon.

8. Verfahren nach einem der vorhergehenden Ansprüche, wobei das Substrat 2-[2-Benzthiazoyl]-6'-hydroxybenthiazolphosphat, 2'-[2-Benzthiazoyl]-6'-hydroxybenzthiazolphosphat-bis-[2-amino-2-methyl-1,3-propandiol], 4-MUP, HNPP; 4-MUG, Luminol/4-Iodphenol, DAB, OPD, AEC, 5AS, 2,2-Azino-bis(3-ethylbenzthiazolin-6-sulfonsäure), 4C1N, o-Dianisidin, TMB, ABTS, BCIP, Naphthol-AS-TR-Phosphat, pNPP, PMP, X-Gal oder CPRG ist.

9. Verfahren nach einem der vorhergehenden Ansprüche, wobei der Katalysator ein Enzym ist.

10. Verfahren nach Anspruch 9, wobei der Katalysator aus der Gruppe ausgewählt ist, die aus Folgenden besteht: Phosphatase, alkalische Phosphatase, β-Galactosidase, Peroxidase, Meerrettichperoxidase, β-Glucuronidase, β- Glucose-6-phosphat-Dehydrogenase, GlucoseOxidase, Urease, Luciferase, β-Lactamase und β-Amylase.

11. Verfahren nach einem der vorhergehenden Ansprüche, wobei wenigstens ein erhaltenes Produkt photolumineszent oder chemilumineszent ist.

12. Verfahren nach einem der vorhergehenden Ansprüche, wobei wenigstens ein erhaltenes Produkt fluoreszent ist.

13. Verfahren nach einem der vorhergehenden Ansprüche, wobei wenigstens ein erhaltenes Produkt elektromagnetische Strahlung im Bereich von 300 nm bis 1.200 nm emittiert, wenn das Produkt einer elektromagnetischen Strahlung im Bereich von 250 nm bis 600 nm ausgesetzt ist.

14. Verfahren nach einem der vorhergehenden Ansprüche, wobei wenigstens ein erhaltenes Produkt im Vorfeld des Aufzeichnens eines Bildes durch Anregungslicht angeregt wird.

15. Verfahren nach Anspruch 3, wobei wenigstens eine Kopplung zwischen dem Katalysator und der Zelle einen oder mehrere Antikörper umfasst.

16. Verfahren nach Anspruch 3, wobei wenigstens ein Katalysator zu einem Antikörper, welcher immunologisch an ein Antigen in der Zellspezies gebunden ist, konjugiert ist.

17. Verfahren nach Anspruch 16, wobei wenigstens ein Katalysator zu einem immunologisch an den Zweitantikörper gebundenen Erstantikörper, welcher Zweitantikörper immunologisch an ein Antigen in der Zellspezies gebunden ist, konjugiert ist.

18. Verfahren nach Anspruch 2, wobei wenigstens ein Katalysator zu Avidin konjugiert ist.

19. Verfahren nach Anspruch 2, wobei wenigstens ein Katalysator zu Streptavidin konjugiert ist.

20. Verfahren nach einem der vorhergehenden Ansprüche, weiter den Schritt des Entfernens von überschüssigem Katalysator, der nicht mit der Zellenspezies verbunden ist, umfassend.

21. Verfahren nach einem der vorhergehenden Ansprüche, wobei der Schritt des Herstellens eines Produkts in einer flüssigen Umgebung ausgeführt wird.

22. Verfahren nach einem der vorhergehenden Ansprüche, wobei das Bildaufzeichnen die Verwendung eines Konfokalscanners umfasst.

23. Verfahren nach einem der vorhergehenden Ansprüche, wobei das Produktbild unter Verwendung einer Anordnung von Detektorelementen aufgezeichnet wird.

24. Verfahren nach Anspruch 23, wobei das Produktbild unter Verwendung einer zweidimensionalen Anordnung von Detektorelementen aufgezeichnet wird.

25. Verfahren nach Anspruch 23, wobei das Produktbild unter Verwendung eines CCD, eines CMOS, einer Videokamera oder einer Photonenzählkamera aufgezeichnet wird.

26. Verfahren nach einem der vorhergehenden Ansprüche, wobei das Bild ohne Vergrößerung aufgezeichnet wird.

27. Verfahren nach einem der vorhergehenden Ansprüche, wobei das Bild mit einem Vergrößerungsfaktor unter 20 aufgezeichnet wird.

28. Verfahren nach einem der vorhergehenden Ansprüche, wobei das Bild in einer Aufnahme aufgezeichnet wird.

29. Verfahren nach einem der Ansprüche 1-27, wobei das Bild in zwei, drei oder mehr Aufnahmen aufgezeichnet wird.

30. Verfahren nach einem der vorhergehenden Ansprüche, wobei das aufgezeichnete Bild anhand von Datenverarbeitungsmitteln verarbeitet wird.

31. Verfahren nach Anspruch 30, wobei die Datenverarbeitungsmittel teilweise überlappende Produktbereiche unterscheiden.

32. Verfahren nach einem der vorhergehenden Ansprüche, wobei die Korrelation eine Schätzung der Punktezahl auf dem Bild umfasst.

33. Verfahren nach einem der vorhergehenden Ansprüche, wobei die Korrelation eine Schätzung der Punktegröße auf dem Bild umfasst.

34. Verfahren nach einem der vorhergehenden Ansprüche, wobei die Korrelation eine Unterscheidung zwischen wenigstens zwei spektralen Produkteigenschaften umfasst.

35. Verfahren nach einem der vorhergehenden Ansprüche, wobei der wenigstens eine Quantitätsparameter aus der Gruppe ausgewählt ist, die aus Viabilität, Größe, Identität, Atmung und Vorhandensein der Zelle besteht.

36. Verfahren nach einem der vorhergehenden Ansprüche, wobei der wenigstens eine Qualitätsparameter aus der Gruppe ausgewählt ist, die aus Anzahl von Zellspezies in einem Probenvolumen, Konzentration von Zellspezies in einem Probenvolumen, Menge von Zellspezies in einem Probenvolumen besteht.

## Revendications

1. Procédé d'évaluation d'au moins un paramètre de qualité ou d'au moins un paramètre de quantité d'au moins une espèce de cellules dans un échantillon de liquide comprenant les étapes de
l'établissement d'un compartiment d'échantillons présentant au moins une paroi,
l'agencement de complexes catalyseur-cellule entre l'au moins une espèce de cellules et au moins un catalyseur dans le compartiment d'échantillons d'une manière permettant aux cellules de se déplacer par rapport à la(aux) paroi(s) du compartiment d'échantillons, l'agencement d'un substrat dans le compartiment d'échantillons, ledit substrat étant capable d'être transformé en un produit à travers une catalyse par ledit catalyseur,
la mise en contact du substrat avec les complexes catalyseur-cellule de cellules individuelles, permettant la production d'une quantité détectable de taches de produits, le produit se diffusant à partir des cellules pour former des taches de forme sensiblement sphérique autour des cellules,
l'enregistrement d'une image des taches de produit relatives aux cellules individuelles dans le compartiment d'échantillons après la production d'une quantité détectable de produit et avant la convergence des différentes taches, et
la mise en corrélation de l'image avec l'au moins un paramètre de qualité ou au moins un paramètre de quantité de l'au moins une espèce de cellules.

2. Procédé selon la revendication 1, dans lequel le complexe catalyseur-cellule comprend une liaison sélective de l'espèce.

3. Procédé selon la revendication 2, dans lequel la liaison sélective de l'espèce comprend une liaison antigène-anticorps.

4. Procédé selon la revendication 1, l'au moins une espèce de cellule étant un marqueur médical d'une maladie.

5. Procédé selon la revendication 1, dans lequel les cellules sont choisies parmi les cellules de mammifère, les cellules d'insectes, les cellules de reptile, les cellules de poisson, les cellules de levure et les cellules fongiques.

6. Procédé selon la revendication 1, dans lequel les cellules sont choisies parmi les cellules sanguines, les cellules spermatiques et les cellules de moelle osseuse.

7. Procédé selon l'une quelconque des revendications précédentes, l'échantillon étant choisi parmi le groupe constitué du lait, des produits laitiers, de l'urine, du sang, du sperme, des sécrétions nasales, des larmes, des fèces, des eaux usées, des eaux de traitement, de l'eau potable, du liquide céphalo-rachidien, de la bile, de la moelle osseuse, des denrées alimentaires, des aliments, et des mélanges, dilutions ou extraits de ceux-ci.

8. Procédé selon l'une quelconque des revendications précédentes, le substrat étant le 2'-[2-benzthiazoyl]-6'-hydroxy-benthiazole phosphate, le 2'-[2-benzthiazoyl]-6'-hydroxy-benthiazole phosphate bis-[2-amino-2-méthyl-1,3-propanediol], le 4-MUP, le HNPP, le 4-MUG, le Luminol/4-iodophénol, le DAB, l'OPD, l'AEC, le 5AS, le 2,2'-azino-bis(3-éthylbenzthiazoline-6-acide sulfonique), le 4C1N, l'o-dianisidine, le TMB, l'ABTS, le BCIP, le Naphthol AS-TR phosphate, le pNPP, le PMP, le X-Gal ou le CPRG.

9. Procédé selon l'une quelconque des revendications précédentes, le catalyseur étant une enzyme.

10. Procédé selon la revendication 9, le catalyseur étant choisi parmi le groupe constitué de la phosphatase, la phosphatase alcaline, la β-galactosidase, la peroxydase, la peroxydase de raifort, la β-glucuronidase, la β-glucose-6-phosphate-déshydrogénase, la glucose-oxydase, l'uréase, la luciférase, la β-lactamase et la β-amylase.

11. Procédé selon l'une quelconque des revendications précédentes, au moins un produit obtenu étant photoluminescent ou chimiluminescent.

12. Procédé selon l'une quelconque des revendications précédentes, au moins un produit obtenu étant fluorescent.

13. Procédé selon l'une quelconque des revendications précédentes, au moins un produit obtenu émettant des rayonnements électromagnétiques dans la plage de 300 nm à 1200 nm lors d'une exposition à des rayonnements électromagnétiques dans la plage de 250 nm à 600 nm.

14. Procédé selon l'une quelconque des revendications précédentes, dans lequel au moins un produit obtenu est excité par une lumière d'excitation avant l'enregistrement d'une image.

15. Procédé selon la revendication 3, au moins une liaison entre le catalyseur et la cellule comprenant deux ou plusieurs anticorps.

16. Procédé selon la revendication 3, au moins un catalyseur étant conjugué à un anticorps lié immunologiquement à un antigène sur l'espèce de cellule.

17. Procédé selon la revendication 16, au moins un catalyseur étant conjugué à un premier anticorps lié immunologiquement à un deuxième anticorps, lié immunologiquement à un antigène sur l'espèce de cellule.

18. Procédé selon la revendication 2, au moins un catalyseur étant conjugué à de l'avidine.

19. Procédé selon la revendication 2, au moins un catalyseur étant conjugué à de la streptavidine.

20. Procédé selon l'une quelconque des revendications précédentes, comprenant en outre l'étape consistant à éliminer l'excédent de catalyseur n'étant pas lié à l'espèce de cellules.

21. Procédé selon l'une quelconque des revendications précédentes, l'étape de la production d'un produit étant réalisée dans un environnement liquide.

22. Procédé selon l'une quelconque des revendications précédentes, l'enregistrement d'image comprenant l'utilisation d'un scanner confocal.

23. Procédé selon l'une quelconque des revendications précédentes, l'image de produit étant enregistrée à l'aide d'un ensemble de dispositifs de détection.

24. Procédé selon la revendication 23, l'image de produit étant enregistrée à l'aide d'un ensemble de dispositifs de détection bidimensionnel.

25. Procédé selon la revendication 23, dans lequel l'image de produit est enregistrée à l'aide d'un CCD, un CMOS, une caméra vidéo ou une caméra à comptage de photons.

26. Procédé selon l'une quelconque des revendications précédentes, l'image étant enregistrée sans grossissement.

27. Procédé selon l'une quelconque des revendications précédentes, l'image étant enregistrée avec un facteur de grossissement inférieur à 20.

28. Procédé selon l'une quelconque des revendications précédentes, l'image étant enregistrée en une exposition.

29. Procédé selon l'une des revendications 1 à 27, l'image étant enregistrée en deux ou trois, ou plus, expositions.

30. Procédé selon l'une quelconque des revendications précédentes, l'image enregistrée étant traitée à l'aide d'un moyen de traitement de données.

31. Procédé selon la revendication 30, le moyen de traitement de données distinguant partiellement les zones de chevauchement de produit.

32. Procédé selon l'une quelconque des revendications précédentes, la mise en corrélation comprenant l'évaluation du nombre de taches sur l'image.

33. Procédé selon l'une quelconque des revendications précédentes, la mise en corrélation comprenant l'évaluation de la taille des taches sur l'image.

34. Procédé selon l'une quelconque des revendications précédentes, la mise en corrélation comprenant la distinction entre au moins deux propriétés spectrales du produit.

35. Procédé selon l'une quelconque des revendications précédentes, l'au moins un paramètre de qualité étant choisi parmi le groupe constitué de la viabilité, la taille, l'identité, la respiration et la présence de la cellule.

36. Procédé selon l'une quelconque des revendications précédentes, l'au moins un paramètre de quantité étant choisi parmi le groupe constitué du nombre d'espèces de cellule présentes dans un volume d'échantillon, de la concentration des espèces de cellule présentes dans un volume d'échantillon et de la quantité d'espèces de cellule présentes dans un volume d'échantillon.
